# EUROPEAN PATENT APPLICATION

(11) **EP 4 309 731 A1**
(43) Date of publication of application: **24.01.2024**
(21) Application number: 23185634.5
(22) Date of filing: 14.07.2023
(51) Int. Cl.: A61N 5/10

(54) **RADIATION INCIDENCE MONITORING METHOD AND SYSTEM**

(30) Priority: 18.07.2022 EP 22185429
(71) Applicant: Vision RT Limited, London N3 2JU (GB)
(72) Inventor: BARRETT, Adrian Roger William, London, N3 2JU (GB); HALE, Gideon Matthew, London, N3 2JU (GB); WAGHORN, Benjamin James, London, N3 2JU (GB)
(74) Representative: Demant

(57) **Abstract**

The present invention relates to a method for monitoring radiation incidence onto an object being prepared for and/or undergoing radiation delivery. The object of providing a cost-efficient monitoring method that allows for monitoring radiation incidence onto an object being prepared for and/or undergoing radiation delivery and for ensuring the radiation beams to be delivered to the correct location as planned during delivery planning and avoiding inadvertent delivery to other areas is solved in that the method comprises emitting a light field onto a surface of an object being prepared for and/or undergoing radiation delivery, the light field at least substantially corresponding to radiation incidence of the radiation delivery; monitoring the light field being reflected from the surface of the object; and processing the monitored light field to determine the radiation incidence of the radiation delivery relative to the object at least partially based on the monitored light field.

## Description

### Technical Field

The present disclosure relates to a method for monitoring radiation incidence onto an object being prepared for and/or undergoing radiation delivery. In addition, the invention relates to a monitoring system for use with a radiation treatment apparatus and radiotherapy systems.

### Background

In radiotherapy, a radiation beam emitted by a radiation source of a radiotherapy apparatus is projected onto a predetermined region of a patient's body in order to destroy or eliminate tumors existing therein as well as treating non-cancerous ailments. Such treatment is usually carried out periodically and repeatedly. At each medical intervention, the radiation source must be positioned with respect to the patient in order to irradiate the selected region with the highest possible accuracy to avoid radiating adjacent tissue on which radiation beams would be harmful.

To this end, radiotherapy usually takes place in two stages. In an initial planning stage, the patient's body is scanned using a 2-D x-ray or a computer tomography (CT) scanner, or by Magnetic Resonance Imaging (MRT) to visualize the target site and any obstacles. The course of therapy is then planned using the images obtained from the x-ray or CT scanner or using the MRI images. Subsequently in a treatment stage, the patient is irradiated in accordance with the course of treatment planned during the planning stage.

A fundamental problem with radiotherapy is the need to position the patient in the same position, when obtaining diagnostic images and each of the subsequent times when radiation is applied to the patient's body. Known radiotherapy apparatuses are calibrated such that the generated radiation beams are focused on what is referred to as the treatment isocenter. The apparatus may include a light field which allows the therapist to visualize the field size and shape, including isocenter location, prior to beam delivery. To ensure accurate delivery to the correct location as planned during treatment planning, the patient lies on a treatment couch which can be positioned in all three directions of space by mechanical drives. It is then the task of a therapist to adjust the position of the couch such that the isocenter of the radiotherapy apparatus coincides with the patient's body region to be treated. The patient's body, or parts of it, can be fixed to the treatment couch to avoid that movements by the patient lead to a depositioning during the treatment session. Moreover, present systems for positioning patients may include various forms of systems for placing markers on the patient to enable the patient to be realigned for different applications of therapy.

Nevertheless, patient motion or setup discrepancies may occur and result in harmful radiation of adjacent healthy tissue. Likely errors may include planning or machine errors, e.g. a multi leaf collimator (MLC) leaf being inadvertently in the incorrect place or misplacement in field match lines, incorrect bolus placement, e.g. resulting in an overspill of the bolus by the beam being delivered directly to the patient's skin rather than through the bolus or inversely if only partial surface coverage by bolus is planned, or changes in patient position, e.g. patient movements or other areas of the body being in the primary beam.

For this reason, monitoring systems for detecting patient movements and assisting the positioning of patients during radiotherapy such as known from European patent application EP 3 294 137 A1 are provided. Such monitoring systems often include cost-intensive stereoscopic cameras and processing modules able to generate 3D patient models for tracking a patient's position.

Also the monitoring system known from European patent application EP 3 294 414 A1 makes use of stereoscopic cameras and processing modules for 3D patient tracking. Moreover, the monitoring necessitates dose delivery to patients prior to detecting delivery anomalies as the monitoring is based on Cherenkov radiation.

Against this background, the present invention faces the object of providing a cost-efficient monitoring method that allows for monitoring radiation incidence onto a patient being prepared for and/or undergoing radiation treatment and for ensuring the radiation beams to be delivered to the correct location as planned during treatment planning and avoiding inadvertent delivery to other areas. In addition, an object of the invention is to specify accordingly improved radiotherapy systems.

### Summary

In accordance with one aspect of the disclosure, there is provided a method for monitoring radiation incidence onto an object being prepared for and/or undergoing radiation delivery, the method comprising:
- emitting a light field onto a surface of an object being prepared for and/or undergoing radiation delivery, the light field at least substantially corresponding to radiation incidence of the radiation delivery;
- monitoring the light field being reflected from the surface of the object; and
- processing the monitored light field to determine the radiation incidence of the radiation delivery relative to the object at least partially based on the monitored light field.

The object can be e.g. a patient or a radiotherapy phantom. When the object is a patient, the radiation delivery can be a radiation treatment.

Therefore, the method according to the first aspect allows for monitoring radiation incidence, in particular where the radiation dose is incident, onto a patient being prepared for and/or undergoing radiation treatment. Radiation treatment may, for example, refer to radiosurgery and/or radiotherapy. In radiosurgery or radiotherapy, intense and precisely collimated doses of radiation are delivered to a target region, e.g. a volume of tumorous tissue, in the body of a patient in order to treat or destroy lesions.

In particular, radiotherapy is a therapy using ionizing radiation, generally provided as part of cancer treatment to control or kill malignant cells. Usually, radiotherapy is delivered by a radiation treatment apparatus such as a linear accelerator. Radiotherapy may be curative in a number of types of cancer, e.g. if they are localized to one area of the body. It may also be used as part of adjuvant therapy, e.g. to prevent tumor recurrence after surgery to remove a primary malignant tumor, e.g. early stages of breast cancer.

Radiosurgery is surgery using radiation. Target regions are precisely destructed using ionizing radiation rather than excision with a blade. Like other forms of radiation therapy, it may usually be used to treat cancer. In stereotactic radiosurgery (SRS), a three-dimensional coordinate system may enable accurate correlation of a virtual target seen in the patient's diagnostic images with an actual target position in the patient.

Prior to radiation treatment, a computer tomography (CT) and/or magnet resonance (MR) scan of the region of interest may be obtained in order to prepare a radiation treatment plan individually tailored to the patient. The planning CT and/or MRT may be used to define the at least one target volume, the so-called target region of interest, and mark organs at risk. Based on these specifications, an individual radiation treatment plan may be developed in which it is precisely specified which areas are to be irradiated with which dose and which regions must be optimally spared. Beam's eye view (BEV)-based planning may improve three-dimensional coverage of a tumor, irradiation techniques, and the therapeutic ratio by decreasing the irradiation of normal tissue.

A precise positioning of the patient, in particular the position and/or orientation of the patient in relation to the radiation beam source, in particular the radiotherapy apparatus, in accordance with the radiation treatment plan, is a prerequisite for actually performing radiation treatment. Thus, prior to radiation treatment, the patient and/or the radiation beam source, in particular the radiotherapy apparatus, have to be set up in order to accurately irradiate the target region of interest. The irradiation of healthy tissue should be avoided. In this context, the method according to the first aspect allows for monitoring radiation incidence for accurate patient set up and/or treatment.

Of course, during radiation treatment, ensuring of the correct radiation incidence is even more crucial due to actual ionizing radiation. During radiation treatment, at least one radiation beam may irradiate the region of interest in the patient, such as a diseased tissue including a tumor or cancerous growth site. In particular, a plurality of radiation beams may be directed to the target region of interest from several positions outside the patient's body. A radiation apparatus may comprise a gantry including a radiation source that may be rotated to provide radiation beams from different positions. At each medical intervention, the radiotherapy apparatus, in particular the radiation source, may be positioned with respect to the patient in order to irradiate the selected region with the highest possible accuracy.

As radiation incidence monitoring allows looking at where the radiation is actually hitting the patient's body, it can highlight errors such as changes in body habitus or posture, for example the patient's arm coming into the radiation field, the radiation unintentionally hitting the chin or the contralateral breast, misalignment of the bolus material, or errors in the delivery system or treatment plan such as erroneous MLC openings and positions.

The method according to the first aspect comprises emitting a light field onto a surface of the object being prepared for and/or undergoing radiation delivery. The light field at least substantially corresponds to radiation incidence of the radiation delivery, in particular at least one radiation beam. Radiation incidence may refer to incidence of at least one radiation beam onto the surface of an object. In particular, monitoring radiation incidence may include monitoring of the position, orientation, size and/or shape of at least one radiation beam. The at least one radiation beam may e.g. be mimicked by at least one beam of visible light causing at least part of the light field. The at least one beam of visible light may hit a surface of the object causing a light field at the region of impact.

For emitting the light field, a radiotherapy apparatus and/or the monitoring system may comprise at least one light field emitting device configured to emit a light field onto a surface of a object being prepared for and/or undergoing radiation delivery. The light field emitting device may, for example, be part of the radiotherapy apparatus, in particular of a gantry of the radiotherapy apparatus. Preferably, the light field emitting device may be positioned close to a beam source of the radiotherapy apparatus. The at least one light field emitting device may be e.g. located before at least one beam-limiting device, e.g. field jaws and/or a multi leaf collimator, of a radiotherapy apparatus, in particular of a linear accelerator head, so that the current shape of the at least one beam-limiting device may be seen on a treatment couch or any object on the couch. Potentially, an already existing light field emitting device in a radiotherapy apparatus, e.g. a linear accelerator, may allow for visualizing radiation incidence, in particular the field size and shape, preferably including isocenter location, prior to and/or during beam delivery. This would be particularly cost-efficient as no additional hardware would be required.

The light field emitted onto the surface of the object may be based on visible light, i.e. electromagnetic radiation within the portion of the electromagnetic spectrum that is perceived by the human eye. In particular, the light has a wavelength in the range of 400 to 700 nm. This allows the light field to be perceived by any monitoring device configured to monitor visible light, in particular a conventional camera, for example.

In particular, prior to an actual radiation delivery (for example, radiation treatment, e.g. during set up of the patient), the light field may represent radiation incidence as it would be during actual beam delivery. The light field may thus represent radiation incidence prior to and/or during actual beam delivery. In this way, conclusions may be drawn about the radiation beam, in particular as at least one radiation beam may be mimicked with visible light.

The method further comprises monitoring the light field being reflected from the surface of the object. In particular, the light field is monitored prior to and/or during radiation delivery. By monitoring the light field, radiation incidence may be monitored prior to and/or during actual beam delivery of a radiation delivery. For example, the monitored light field allows for real-time conclusions of radiation incidence throughout a radiation delivery and preferably ensuring accuracy through the entire delivery process.

For monitoring the light field, at least one monitoring device configured to monitor the light field may be provided. Preferably, the at least one monitoring device is at least one remote monitoring device. In general, any monitoring device configured to monitor the light field, in particular configured to monitor visible light emitted onto a surface, including a conventional camera or the like, is conceivable. For example, at least one stereoscopic camera may be provided. By at least one stereoscopic camera, it may be achieved that using the principles of stereoscopy, visual data enabling a three-dimensional representation of e.g. the light field and/or the object is provided. The at least one monitoring device may preferably be arranged in the treatment room for monitoring the light field emitted onto the surface of the object. The at least one monitoring device may be calibrated to the isocenter of the treatment apparatus, e.g. the isocenter of the radiotherapy apparatus.

The at least one monitoring device may comprise at least one filter configured to selectively transmit light, preferably of at least one particular wavelength, in particular a defined range of wavelengths in the spectrum of visible light, i.e. between 400 to 700 nm. This may be particularly advantageous if the method according to the first aspect and/or the monitoring system according to the second aspect is used in combination with another non-contact motion detection system such as Vision RT's AlignRT^{®} system as is disclosed in the international patent application WO 2004/004828 A2. For example, in the Vision RT's AlignRT^{®} system, speckle projections may be given by AlignRT camera pods. A challenge in monitoring the light filed may be to distinguish the light field, which preferably is based on visible light, from other sources of illumination in the scene. Monitoring the light field being reflected from the surface of the object may thus comprise filtering the light for selectively transmitting light of at least one particular wavelength. This is particularly advantageous in case a different monitoring device is used for the Vision RT's AlignRT^{®} system, in particular for monitoring the speckle projections, than for monitoring the light field in accordance with the first and/or second aspect of the present disclosure. In case the same monitoring device is used for monitoring the light field and the speckles, it is conceivable that distinguishing the light field may be achieved by strobing the speckle and monitoring, in particular acquiring images, both with the speckle, e.g. for standard AlignRT 3D surface acquisition, and without the speckle, in particular for light filed monitoring in accordance with the first and/or second aspect of the present disclosure. External light sources such as room lighting are preferably lowered when monitoring the light field so as to at least reduce competing sources of light.

The monitoring device typically provide a 2D or 3D image showing the light field incidence on the surface of the object, viewed from the position of the monitoring device.

The monitoring system can obtain or generate a reference image showing a reference light field incidence on the surface of the object, using a defined radiation delivery plan (such as a radiation treatment plan when the object is a patient), e.g. parameters obtained from the treatment plan or from the treatment system. The reference image is typically a 2D or a 3D virtual image.

For example, a DICOM reference surface of the object, obtained from CT scanner, a predetermined position of the gantry and/or a predetermined rotation of the collimator, and/or a predetermined position of the MLC leaves can be used to generate the 2D or 3D virtual image.

Instead of a DICOM reference surface of the object, a reference surface captured by the stereoscopic camera or the non-contact motion detection system, or an updated reference surface can be used, the updated reference surface being updated with the current measured position of the object as measured by the ROI (Region Of Interest) monitoring of the non-contact motion detection system.

Alternatively, the reference image may be generated directly by the treatment planning system used to prepare the radiation treatment plan as a dose distribution or dose outline.

Objects being set up for and/or undergoing radiation delivery are typically placed on a mechanical treatment couch of a radiotherapy treatment system. Preferably, monitoring of the radiation incidence may include monitoring of the position and/or orientation of a radiotherapy apparatus relative to the object, in particular a treatment couch holding the object. This allows for drawing more precise conclusions about the incidence of the at least one radiation beam.

The method according to the first aspect further comprises processing the monitored light field to determine the radiation incidence of the radiation delivery relative to the object at least partially based on the monitored light field. For processing the monitored light field, at least one processing unit configured to process the monitored light field to determine the radiation incidence of the radiation delivery relative to the object at least partially based on the monitored light field may be provided. The at least one processing unit may be an internal processing unit of a monitoring system, or it may be an external processing unit, for example a general-purpose personal computer that is connected by wires or wirelessly to the at least one monitoring device and that runs a respective computer program, or a combination of both.

The at least one processing unit may be an internal processing unit of the monitoring system, or it may be an external processing unit, for example a general-purpose personal computer that is connected by wires or wirelessly to the at least one monitoring device and that runs a respective computer program, or a combination of both. For example, the processing unit is a remote processing unit. The remote processing unit may be located in a room different from the treatment room.

For example, when the monitoring device provides an image of the light field incidence and the monitoring system obtains or generates a reference image including a reference light field incidence, metrics can be used to compare the image of the light field incidence with the reference image, and to provide quantitative measurements about how the light field incidence differs from the reference light field incidence, and therefore how the determined radiation incidence of the radiation delivery differs or deviates from a predetermined radiation incidence.

Qualitative means can be used to show important information to the therapist. For example, a colour can be used to highlight the detected light field incidence on the patient. For example, a blue colour can be overlaid on a greyscale video image of the object to show where the light field is falling. A first outline can be used to show where the detected light field is, and a second outline, based on the reference light field incidence, can be used to show where it should be, to help the therapist to assess whether the radiation beam is delivered to the correct location.

During a radiation delivery, the gantry and or the MLC can move and therefore the shape of the light field can vary from moment to moment. Thus, in order to have a global monitoring of radiation incidence, the monitoring device can provide a set of images of the light field incidence, each image showing the light field incidence at several predefined times of the radiation delivery, and the monitoring system can obtain or generate a set of reference images including a reference light field incidence at the same several predefined times of the radiation delivery.

The set of images of the light field incidence is then used to generate a cumulative image of the light field incidence, typically showing where light field has been detected during the delivery. For example, each image of the light field incidence can be a binary image (either the light field is present in a pixel or it is not). In such a case, when the images of the set of images of the light field incidence are added together, a cumulative image showing where light field has been detected in the course of the delivery can be obtained. Alternatively, each image of the light field incidence can be a grayscale image.

Moreover, the set of reference images including a reference light field incidence can be used to generate a cumulative reference image. The cumulative image of the light field incidence can be then compared to the cumulative reference image e.g. to retrospectively compare the radiation incidence to the planned delivery.

Preferably, the method according to the first aspect further includes performing an action, e.g. adjusting parameters or inhibiting radiation, based on the determined radiation incidence. For example, in the event of deviations from the predetermined radiation incidence, delivery parameters may be adjusted. This may include, for example, (re)positioning of the object, a treatment couch holding the object, a radiotherapy apparatus and/or modifying the delivery plan. The ability to deliver the radiation dose to the target region correctly may depend on several factors, including radiation spot location, as well as knowledge of the precise object geometry used during irradiation. The factors influencing accuracy of radiation beam delivery may also depend on the mechanical precision and movements of the apparatus itself and of the apparatus and/or treatment accessories such as bolus, wedges, blocks, etc. There are numerous parameters, e.g. beam alignment, beam symmetry, beam shape, and beam flatness, as well as field shape and width, associated with a radiation therapy system that may influence the accuracy of the radiation incidence.

The method according to the first aspect allows for safe and non-invasive monitoring of radiation incidence onto a patient being prepared for and/or undergoing radiation treatment. It has been recognized in the context of the method according to the first aspect that by monitoring the light field being reflected from the surface of the patient and processing the monitored light field to determine the radiation incidence of the radiation treatment relative to the patient at least partially based on the monitored light field, the radiation incidence of a radiation beam prior to and/or during radiation treatment can be monitored and eventually corrected. Thus, a safe, non-invasive method for tracking radiation incidence, in particular relative to a patient's position and/or to a radiotherapy apparatus, before and during radiotherapy is provided, to help ensuring a streamlined workflow for accurate treatment delivery.

The method according to the first aspect allows for a fast and accurate patient setup and monitoring for all forms of cancer, including breast, brain, lung, liver, sarcoma, head and neck, and other cancers. In particular, with the method according to the first aspect of the disclosure radiation beams to be delivered to the correct location as planned during treatment planning and avoiding inadvertent delivery to other areas may be ensured. On top of that, also bolus placement, e.g. for reducing or altering dosing, may be simplified.

Moreover, the method according to the first aspect may reduce patient treatment time due to less repeat imaging and more accurate initial setup. The light field may be assessed during patient setup, i.e. prior to beam delivery, or as part of a plan or system quality assurance program. On top of that, the method according to the first aspect may also benefit patient comfort, as it may eliminate the need for tattoos or other permanent marks and/or avoid the use of traditional closed mask or frame-based SRS, and even reduce the need for pediatric anesthesia.

A second aspect of the present disclosure relates to a monitoring system for use with a radiotherapy apparatus to monitor radiation incidence onto an object being prepared for and/or undergoing radiation delivery, in particular for performing a method to one of the previous claims. The monitoring system comprises:
- at least one light field emitting device configured to emit a light field onto a surface of an object being prepared for and/or undergoing radiation delivery, the light field at least substantially corresponding to radiation incidence of the radiation delivery;
- at least one monitoring device configured to monitor the light field being reflected from the surface of the object; and
- at least one processing unit configured to process the monitored light field to determine the radiation incidence of the radiation delivery relative to the object at least partially based on the monitored light field.

A third aspect of the present disclosure relates to a radiotherapy treatment system. The radiotherapy treatment system comprises:
- a radiotherapy apparatus operable to irradiate an object with radiation; and
- a radiation incidence monitoring system according to the second aspect.

The object can be e.g. a patient or a calibration phantom. When the object is a patient, the radiation delivery can be a radiation treatment.

Exemplary embodiments of the first, second, and third aspect of the disclosure may have one or more of the properties described below.

Determining the radiation incidence of the radiation treatment relative to the patient may include determining the radiation incidence of the radiation treatment relative to a predetermined radiation incidence in accordance with a defined radiation treatment plan.

A radiation treatment plan may be individually tailored to the patient. In radiation treatment planning, a planning CT may be used to define a target region of interest and mark organs at risk. Based on these specifications, an individual radiation treatment plan may be developed in which it is precisely specified which areas are to be irradiated with which dose and which regions must be optimally spared. Preferably, the radiation treatment plan may indicate a predetermined radiation incidence. Determining the radiation incidence of the radiation treatment relative to the predetermined radiation incidence in accordance with the defined radiation treatment plan allows for ensuring the radiation beams to be delivered to the correct location as planned during treatment planning and thus avoiding inadvertent delivery to other areas.

The method may further comprise triggering an alarm (or a warning) if the determined radiation incidence of the radiation delivery differs from the predetermined radiation incidence determined from the radiotherapy delivery plan (which can be a radiotherapy treatment plan when the object is a patient) by more than a threshold amount. An alarm may include an audible, visual and/or other kind of signal to alert e.g. a therapist to the deviation in radiation incidence. The threshold amount may, for example, be specified in delivery planning or treatment planning and/or be dependent on the type of treatment and/or region of interest. For example, it is conceivable that the threshold amount may be an absolute value, e.g. expressed in millimeters, or a relative amount, e.g. expressed in a percentage.

Accordingly, the radiotherapy apparatus may be operable to trigger an alarm and/or to inhibit or trigger inhibition of irradiation of the object if the determined radiation incidence of the radiation delivery relative to the object differs from a predetermined radiation incidence determined from a radiotherapy delivery plan by more than a threshold amount.

The method may further comprise inhibiting or triggering inhibition of irradiation of the object if the determined radiation incidence of the radiation delivery differs from the predetermined radiation incidence determined from the radiotherapy delivery plan by more than a threshold amount. Ensuring of the correct radiation incidence is particularly crucial during radiation treatment. During radiation treatment, deviation from a predetermined radiation incidence by more than a predefined threshold amount may e.g. cause a signal to be provided to the radiotherapy apparatus to pause irradiation. This allows for ensuring that radiation is only delivered when the object (e.g. the patient) is positioned relative to the radiation beam as desired.

Deviation from a predetermined radiation incidence may, for example, be caused by movements of the patient, e.g. during breast treatment, the patient's arm or chin may be in the beam and thus there may be a risk of dose delivery to the wrong area. But also, for example, a variation in breathing of the patient may cause deviation from the predetermined radiation incidence. In such a case, a signal may be provided to the radiotherapy apparatus to pause irradiation.

It is conceivable that irradiation may only be inhibited as long as the determined radiation incidence of the radiation delivery differs from the predetermined radiation incidence determined from the radiotherapy delivery plan by more than the threshold amount and irradiation may be continued in case the deviation remains below the threshold.

Moreover, there may be at least a first and a second threshold amount provided. In particular, the method may comprise triggering an alarm if the determined radiation incidence of the radiation delivery differs from the predetermined radiation incidence determined from the radiotherapy delivery plan by more than a first, in particular lower, threshold amount and inhibiting or triggering inhibition of irradiation of the object if the determined radiation incidence of the radiation delivery differs from the predetermined radiation incidence determined from the radiotherapy delivery plan by more than a second, in particular higher, threshold amount. It is conceivable that either action, i.e. triggering an alarm and/or inhibiting or triggering inhibition of irradiation, is only performed as long as the respective threshold is exceeded.

The method may further comprise positioning or triggering positioning of the object being prepared for and/or undergoing radiation delivery, in particular of a couch holding the object, based on the determined radiation incidence of the radiation delivery relative to the object. Particularly, the object, in particular the treatment couch holding the object, may be positioned depending on the determined radiation incidence. It is conceivable that the object is repositioned in case the radiation incidence deviates from the radiation incidence according to a predetermined radiation incidence in accordance with a radiation delivery plan. In particular the object may be repositioned in case the deviation exceeds a certain threshold amount.

Preferably, a radiotherapy treatment system according to the third aspect may further comprise a couch for positioning the object being prepared for and/or undergoing radiation delivery relative to the radiotherapy apparatus, in particular a gantry of the radiotherapy apparatus. The couch and/or the radiotherapy apparatus, in particular the gantry of the radiotherapy apparatus, may be configured to change the position and/or orientation depending on the determined radiation incidence of the radiation delivery relative to the object.

The method may further comprise positioning or triggering positioning of a radiotherapy apparatus for irradiation of the object, in particular of a gantry of the radiotherapy apparatus, based on the determined radiation incidence of the radiation delivery relative to the object. It is conceivable that the radiation treatment apparatus is repositioned in case the radiation incidence deviates from the radiation incidence according to a predetermined radiation incidence in accordance with a radiation delivery plan. In particular the radiotherapy apparatus may be repositioned in case the deviation exceeds a certain threshold amount.

For example, the treatment couch and the radiotherapy apparatus may be arranged such that the relative positions may be varied, e.g. laterally, vertically, longitudinally and/or rotationally. Positioning of the at least one treatment couch and/or the at least one radiotherapy apparatus may, for example, be caused by at least one processing unit.

Positioning or triggering positioning of the object being prepared for and/or undergoing radiation delivery and/or the radiotherapy apparatus for irradiation of the object may be performed iteratively. For example, the method according to the first aspect may, for example, comprise monitoring of respiratory motion by the patient prior to and/or during radiation treatment. Positioning or triggering positioning of the patient may be performed iteratively based on the monitored light field and respiratory motion by the patient prior to and/or during radiation treatment.

Determining the radiation incidence of the radiation delivery relative to the object may include determining the position, orientation and/or shape of at least one radiation beam relative to the object. Conclusions about the at least one radiation beam may be drawn, for example, by processing the monitored light field. Alternatively or additionally, the method may comprise monitoring of the position and/or orientation of a treatment couch holding the object and/or of at least one radiotherapy apparatus, in particular of a gantry of at least one radiotherapy apparatus, relative to an object being irradiated. Determining the position, orientation and/or shape of at least one radiation beam relative to the object may be performed based on the monitored position and/or orientation of the treatment couch holding the object and/or of the at least one radiotherapy apparatus relative to an object being irradiated.

Moreover, the method may alternatively or additionally comprise monitoring of the position, orientation, size and/or shape of at least one radiation beam source and/or beam-limiting device, in particular field jaws and/or a collimator such as a MLC, of at least one radiotherapy apparatus. An MLC is a beam-limiting device that is made of individual leaves of a high atomic numbered material, usually tungsten, that may be moved independently in and out of the path of a radiation beam in order to shape it and vary its intensity. This allows for quick and easy beam shaping and is particularly cost-effective and time saving.

The monitored position, orientation, size and/or shape of the at least one radiation beam source and/or beam-limiting device may, for example, be taken into account when determining the position, orientation and/or shape of at least one radiation beam relative to the object. These embodiments allow for even more precise conclusions about the radiation incidence of the at least one radiation beam to be drawn.

The at least one monitoring device configured for monitoring the light field may also be configured to monitor the position and/or orientation of a treatment couch holding the object and/or of at least one radiotherapy apparatus, in particular of a gantry of at least one radiotherapy apparatus, relative to the object and/or the position, orientation, size and/or shape of at least one radiation beam source and/or beam-limiting device, in particular field jaws and/or MLC, of at least one radiotherapy apparatus. Alternatively or additionally, at least one additional monitoring device may be provided being configured to monitor the position and/or orientation of a treatment couch holding the object and/or of at least one radiotherapy apparatus, in particular of a gantry of at least one radiotherapy apparatus, relative to the object and/or the position, orientation, size and/or shape of at least one radiation beam source and/or beam-limiting device, in particular field jaws and/or MLC, of at least one radiotherapy apparatus.

The at least one processing unit may be configured to determine the shape, size and/or orientation of at least one radiation beam from the position and/or orientation of a treatment couch holding the object and/or of the at least one treatment apparatus relative to an object being irradiated and/or the position, orientation, size and/or shape of at least one radiation beam source and/or beam-limiting device, in particular field jaws and/or an MLC, of at least one radiotherapy apparatus.

The method may further comprise adjusting or triggering adjustment of the position, orientation, size and/or shape of the at least one radiation beam based on the determined radiation incidence of the radiation delivery relative to the object. The method may, for example, comprise adjusting or triggering adjustment of the position and/or orientation of the at least one radiation beam by varying the position and/or orientation of at least one treatment couch holding the object and/or of at least one radiation therapy apparatus for irradiating the object, in particular a gantry of the at least one radiation apparatus. Additionally or alternatively, the method may comprise adjusting or triggering adjustment of the size and/or shape of the at least one radiation beam by varying at least one beam-limiting device such as e.g. field jaws and/or an MLC.

The radiotherapy apparatus of the radiotherapy treatment system according to the third aspect may, for example, be configured to adjust the position, orientation, size and/or shape of at least one radiation beam based on the determined radiation incidence of the radiation delivery relative to the object. To this end, the radiotherapy apparatus may e.g. comprise at least one least one beam-limiting device such as e.g. field jaws and/or an MLC.

The disclosure of a method step is to be understood to also disclose the respective means for performing the method step. Likewise, the disclosure of means for performing a method step is to be understood to also disclose the respective method step.

Further configurations and advantages of the invention will be explained in the following detailed description of some exemplary embodiments of the present invention in conjunction with the drawing.

### Brief description of the drawing

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the drawing described hereinafter in which:

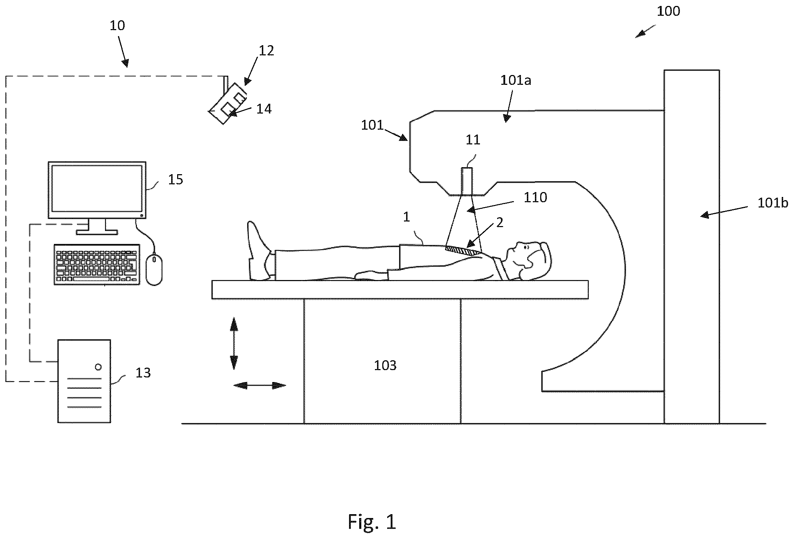

- Fig. 1: is a schematic perspective view of a radiotherapy system according to the third aspect comprising a monitoring system according to the second aspect of the present disclosure.

### Detailed description of the drawing

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

In Fig. 1, a monitoring system 10 according to the second aspect is shown together with a radiotherapy apparatus 101, e.g. a linear accelerator, both together forming a radiotherapy system 100 according to the third aspect. The radiotherapy apparatus 101 comprises a radiation beam source, indicated illustratively by reference numeral 110 in figure 1, which is directed onto a patient 1 for a radiotherapy treatment session. The patient 1 lies on a mechanical treatment couch 103 which can be moved in all three directions of space, two of which being indicated by the arrows in Fig. 1. Additionally, the couch 103 may be rotated in all three directions of space (not shown). The mechanical treatment couch 103 is provided as part of the radiotherapy treatment system 100 upon which a patient 1 lies during treatment. The radiotherapy apparatus 101 and the mechanical treatment couch 103 are arranged such that, under the control of the computer 15, the relative positions of the treatment couch 103 and the radiotherapy apparatus 101 may be varied, laterally, vertically, longitudinally and rotationally as is indicated in the figure by the arrows adjacent the couch 103.

The radiotherapy apparatus 101 comprises a main body 101b from which extends a gantry 101a. A beam-limiting device (not shown) such as field jaws and/or a multi leaf collimator (MLC) is provided at the end of the gantry 101a remote from the main body 101b of the radiotherapy apparatus 101. To vary the angles at which radiation irradiates a patient 1, the gantry 101a, under the control of a processing unit 12, is arranged to rotate about an axis passing through the center of the main body 101b of the radiotherapy apparatus 101. Additionally, the location of irradiation by the radiotherapy apparatus 101 may also be varied by rotating the beam-limiting device at the end of the gantry 101a.

The radiotherapy treatment system 100 further comprises comprises a light field emitting device 11, which in this example is mounted to the radiotherapy apparatus 101, in particular the gantry 101a, but could also be place together with the monitoring system 10. The light emitting device 11 emits a light field onto a surface of a patient 1 being prepared for and/or undergoing radiation treatment, the light field 2 at least substantially corresponding to radiation incidence of the radiation treatment.

The monitoring system 10 further comprises a monitoring device 12, such as a conventional and/or stereotactic camera, as well as a processing unit 13. The monitoring device 12 is connected by wiring (not shown) to a computer 15 comprising the at least one processing unit 13. The monitoring device 12 monitors the light field 2 being reflected from the surface of the patient 1. In order to distinguish the light field 2 from other sources of illumination in the scene, the monitoring device 12 comprises at least one filter 14 configured to selectively transmit light of at least one particular wavelength. The light field 2 monitored with the monitoring device 12 is processed by the processing unit 13 to determine the radiation incidence of the radiation treatment relative to the patient 1 at least partially based on the monitored light field 2.

In use, the camera obtains video images of the light field 2 emitted onto a patient 1 lying on the treatment couch 103. These video images are passed via the wiring to the computer 15. The processing unit 13 then processes the video images of the patient 1 to determine radiation incidence onto the patient 1.

In addition to monitoring the light field 2 emitted onto the surface of the patient 1, the monitoring device 12 is arranged to monitor the position and orientation of the radiotherapy apparatus 101 and/or the treatment couch 103 to determine the orientation of an emitted radiation beam 110 emitted by the radiotherapy apparatus 101 relative to the patient 1. This may be achieved by, for example, monitoring the positions of a number of markers (not shown) on the surface of the radiotherapy apparatus 101.

The projection of the monitored light field 2 on the surface of the patient 1 may be compared to the predetermined radiation incidence based on a radiation treatment plan. If the monitored light field 2 and expected radiation incidence do not match, this may indicate an error in set up and treatment can be inhibited, e.g. paused. Alternatively or additionally, an alarm may be triggered.

For example, during initial set up of the patient prior to radiation treatment, based on the monitored light field 2 and the therewith determined radiation incidence of the radiation treatment relative to the patient 1, positioning or triggering positioning of the patient 1 being prepared for and/or undergoing radiation treatment, in particular of the treatment couch 103 holding the patient, is performed. Positioning instructions necessary to align the actual to the predetermined radiation incidence may be determined and sent to the treatment couch 103. Subsequently, during treatment, any deviation from the initial set up may be identified and if the deviation is greater than a threshold, the computer 15 sends instructions to the treatment couch 103 and/or the radiotherapy apparatus 101 for repositioning the patient 1 in relation to the radiation beam 110 or e.g. to cause treatment to be halted until the patient 1 can be repositioned.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects. Reference to an element in the singular is not intended to mean "one and only one" unless specifically so stated, but rather "one or more." Unless specifically stated otherwise, the term "some" refers to one or more.

Accordingly, the scope should be judged in terms of the claims that follow.

## Claims

1. Method for monitoring radiation incidence onto an object being prepared for and/or undergoing radiation delivery, the method comprising:
- emitting a light field onto a surface of an object being prepared for and/or undergoing radiation delivery, the light field at least substantially corresponding to radiation incidence of the radiation delivery;
- monitoring the light field being reflected from the surface of the object; and
- processing the monitored light field to determine the radiation incidence of the radiation delivery relative to the object at least partially based on the monitored light field.

2. The method according to claim 1,
**characterised in that**
determining the radiation incidence of the radiation delivery relative to the object includes determining the radiation incidence of the radiation delivery relative to a predetermined radiation incidence in accordance with a defined radiation delivery plan.

3. The method according to claim 2,
**characterised in that**
the method further comprises:
- triggering an alarm if the determined radiation incidence of the radiation delivery differs from the predetermined radiation incidence determined from the radiation delivery plan by more than a threshold amount.

4. The method according to claim 2 or 3,
**characterised in that**
the method further comprises:
- inhibiting or triggering inhibition of irradiation of the object if the determined radiation incidence of the radiation delivery differs from the predetermined radiation incidence determined from the radiation delivery plan by more than a threshold amount.

5. The method according to one of claims 1 to 4,
**characterised in that**
the method further comprises:
- positioning or triggering positioning of the object being prepared for and/or undergoing radiation delivery, in particular of a couch holding the object, based on the determined radiation incidence of the radiation delivery relative to the object.

6. The method according to one of claims 1 to 5,
**characterised in that**
the method further comprises:
- positioning or triggering positioning of a radiotherapy apparatus for irradiation of the object, in particular of a gantry of the radiotherapy apparatus, based on the determined radiation incidence of the radiation delivery relative to the object.

7. The method according to claim 5 or 6,
**characterised in that**
the positioning or triggering positioning of the object being prepared for and/or undergoing radiation delivery and/or the radiotherapy apparatus for irradiation of the object is performed iteratively.

8. The method according to one of claims 1 to 7,
**characterised in that**
determining the radiation incidence of the radiation delivery relative to the object includes determining the position, orientation and/or shape of at least one radiation beam relative to the object.

9. The method according to claim 8,
**characterised in that**
the method further comprises:
- adjusting or triggering adjustment of the position, orientation, size and/or shape of the at least one radiation beam based on the determined radiation incidence of the radiation delivery relative to the object.

10. Monitoring system for use with a radiotherapy apparatus to monitor radiation incidence onto an object being prepared for and/or undergoing radiation delivery, in particular for performing a method to one of the previous claims, the monitoring system comprising:
- at least one light field emitting device configured to emit a light field onto a surface of an object being prepared for and/or undergoing radiation delivery, the light field at least substantially corresponding to radiation incidence of the radiation delivery;
- at least one monitoring device configured to monitor the light field being reflected from the surface of the object; and
- at least one processing unit configured to process the monitored light field to determine the radiation incidence of the radiation delivery relative to the object at least partially based on the monitored light field.

11. Monitoring system according to claim 10,
**characterised in that**
the at least one monitoring device comprises:
- at least one filter configured to selectively transmit light.

12. Radiotherapy treatment system comprising:
- at least one radiotherapy apparatus operable to irradiate an object with radiation; and
- at least one radiation incidence monitoring system according to claim 10 or 11.

13. Radiotherapy treatment system according to claim 12,
**characterised in that**
the radiotherapy apparatus is operable to trigger an alarm and/or to inhibit or trigger inhibition of irradiation of the object if the determined radiation incidence of the radiation delivery relative to the object differs from a predetermined radiation incidence determined from a radiation delivery plan by more than a threshold amount.

14. Radiotherapy treatment system according to claim 12 or 13,
**characterised in that**
- the radiotherapy treatment system further comprises a couch for positioning the object being prepared for and/or undergoing radiation delivery relative to the radiotherapy apparatus, in particular a gantry of the radiotherapy apparatus,
wherein the couch and/or the radiotherapy apparatus, in particular the gantry of the radiotherapy apparatus, is configured to change the position and/or orientation depending on the determined radiation incidence of the radiation delivery relative to the object.

15. Radiotherapy treatment system according to any of claims 12 to 14:
**characterised in that**
the radiotherapy apparatus is configured to adjust the position, orientation and/or shape of at least one radiation beam based on the determined radiation incidence of the radiation delivery relative to the object.
